(19) Europäisches Patentamt
European Patent Office
Office européen des brevets

(11) Publication number: **0 046 596**
**B1**

# (12) EUROPEAN PATENT SPECIFICATION

(45) Date of publication of patent specification: **27.06.84**

(21) Application number: **81106595.2**

(22) Date of filing: **25.08.81**

(51) Int. Cl.³: **C 07 D 487/04,**
C 12 P 17/18, A 61 K 31/40
//C12R1/465, (C07D487/04, 209/00, 205/00)

## (54) Antibiotic PA-39504-X3, process for its production and pharmaceutical compositions.

(30) Priority: **25.08.80 JP 117182/80**

(43) Date of publication of application:
**03.03.82 Bulletin 82/9**

(45) Publication of the grant of the patent:
**27.06.84 Bulletin 84/26**

(84) Designated Contracting States:
**CH DE FR GB IT LI NL SE**

(56) References cited:
EP - A - 0 005 349
EP - A - 0 017 246
EP - A - 0 030 719

(73) Proprietor: **SHIONOGI & CO., LTD.**
**12, Dosho-machi 3-chome Higashi-ku**
**Osaka 541 (JP)**

(72) Inventor: **Tanaka, Kentaro**
**3-19-12, Furudeai**
**Suita-shi Osaka Pref (JP)**
Inventor: **Kondo, Eiji**
**4-22-18, Ishibashi**
**Ikedo-shi Osaka Pref (JP)**
Inventor: **Matsumoto, Kouichi**
**6-11-72-401, Higashitoyonoka-cho**
**Toyonaka-shi Osaka Pref. (JP)**
Inventor: **Shoji, Jun'ichi**
**1-17-14, Nagaodai**
**Hirakata-shi Osaka Pref. (JP)**
Inventor: **Tsuji, Naoki**
**14-22, Okuike-cho**
**Ashiya-shi Hyogo Pref. (JP)**

(74) Representative: **Vossius Vossius Tauchner Heunemann Rauh**
**Siebertstrasse 4 P.O. Box 86 07 67**
**D-8000 München 86 (DE)**

**EP 0 046 596 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the new antibiotic PA—39504—$X_3$ and its pharmaceutically acceptable salts, a process for producing it, and pharmaceutical compositions containing it.

The antibiotic PA—39504—$X_3$ and its pharmaceutically acceptable salts are useful antibiotics which have a broad antibacterial spectrum and $\beta$-lactamase inhibiting activity.

PA—39504—$X_3$ has the structural formula I

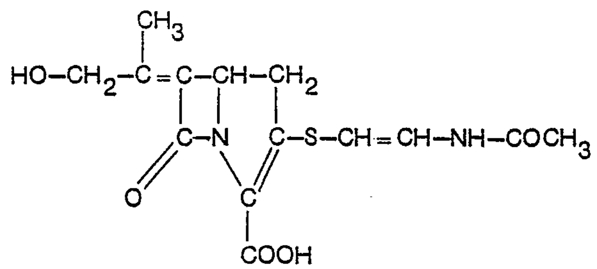

(I)

The present invention includes the above compound PA—39504—$X_3$ and its pharmaceutically acceptable salts. As the salts, the salts with an alkali metal (such as sodium, potassium) and alkaline earth metal (such as calcium, barium) are exemplified.

The sodium salt of PA—39504—$X_3$ has the following physical and chemical properties.

(a) Ultraviolet absorption spectrum:
$\lambda_{max}^{H2O}$ ($E_{1cm}^{1\%}$) nm 234(680), 296(316).

(b) Nuclear magnetic resonance spectrum (Fig. 1)
$\delta_{ppm}^{D2O}$ (J = Hz), 1.96s3H, 2.05s3H, 3.09m2H, 4.21s2H, 4.85m, 6.01d1H (8.4), 7.13d1H (8.4).

(c) Circular dichroism spectrum:
$\lambda_{nm}[\theta]$ 425(O), 350(−2540), 332(−2190), 279(−10600), 263(O), 258(+3030), 252(O), 238(−10900), 224(O), 216.5(+8200), 211(+6060), 200(+23300).

(d) Mass spectrum (Field desorption mass spectrum)
M/z 459 = [$M^+$] (measured as p-nitrobenzyl ester)

In the prior art, epithienamycins and olivanic acids have been known as carbapenem type antibiotics having a $\beta$-lactamase inhibiting activity (P. G. Sammes: Topics in Antibiotic Chemistry, volume 3, 118—123). These compounds, however, are different from PA—39504—$X_3$ in that the substituent in the 6 position is a 1-hydroxyethyl group. It is known from EP—A—30 719 that Streptomyces argenteolus PA—39504 or Streptomyces tokunonensis PA—31088 produces the antibiotic PA—39504—$X_1$ having the formula A

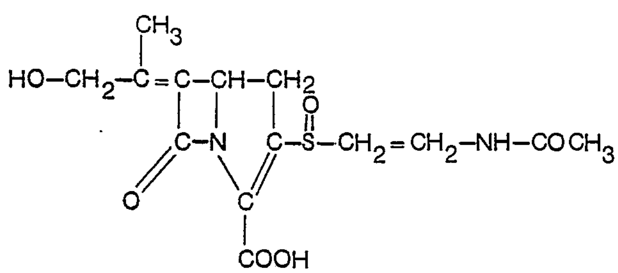

(A)

Furthermore it is known from EP—A—17 246 that Streptomyces tokunonensis PA—31088 produces the antibiotic PA—31008—IV having the formula B

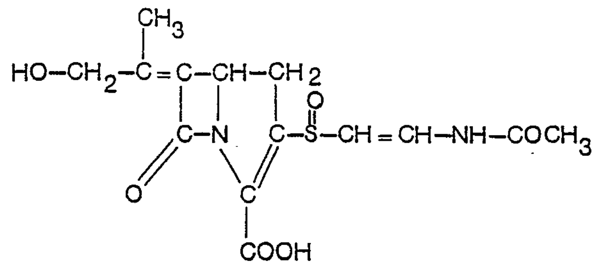

(B)

It has been found that the new antiobiotic PA—39504—$X_3$ is also produced by the aforementioned oraganisms Streptomyces argenteolus PA—39504 and Streptomyces tokunonensis PA—31088. The strain PA—39504 has been deposited with The Agency of Industrial Science & Techonology (Yatabe-machi, Tukuba-gun, Ibaragi Pref.) on Nov. 1, 1979 under the FERM—P No. 5256 and with the American Type Culture Collection (Parklawn Drive, Rockville, Maryland) under the ATCC No. 31589. The strain PA—31088 has been deposited with the Agency of Industrial Science & Technology on February 26, 1979 under the FERM—P No. 4843 and with the American Type Culture Collection under the ATCC No. 31569.

The bacteriological properties of the strains PA—39504 and PA—31088 are characterized as follows.

1. Streptomyces argenteolus PA—30504

(a) Morphological properties (Bennett's agar, 28°C, cultured for 14 days)

This strain grows well on Bennett's agar, and forms aerial hyphae abundantly. The aerial hyphae branch simply, the terminal forming a loop or short spiral. The color of aerial hyphae on the above agar medium is light brownish grey to brownish grey, and that of substrate hyphae is pale yellowish brown. No soluble pigment is produced. The surface structure of the spores are smooth and the spores are short and cylindrical as observed under an electron microscope. Neither sporangium, flagellated spores nor sclerotium are observed, and no split by fragmentation is observed on the substrate hyphae.

(b) Physiological properties

| | |
|---|---|
| Liquefaction of gelatin | negative |
| Prouction of melanoid pigment | negative |
| Tyrosinase reaction | negative |
| Peptonization of milk | positive |
| Coagulation of milk | negative |
| Hydrolysis of starch | positive |

(c) Utilization of sugars

Growth in the presence of:

L-arabinose, D-xylose, D-glucose, D-fructose, inositol, L-rhamnose

No growth observed in the presence of:

sucrose, raffinose, D-mannitol

(d) Growth temperature

10°C Appreciable growth, scarce formation of aerial hyphae

28°C Favourable growth, abundant formation of aerial hyphae

37°C No growth

45°C No growth

2. *Streptomyces tokunonensis* PA—31088

(a) Morphological properties (Bennett's agar, 28°C, cultured for 14 days)

Neither sporangium, flagellated spores nor sclerotium are observed, and no split by fragmentation of substrate hyphae is observed. Aerial hyphae are formed abundantly on the above agar medium. Spore-bearing hyphae are attached to the aerial hyphae, from which simple branches are spread as side branches with spiral terminals. The surface structure of the spores is smooth and the spores are short and cylindrical as observed under an electron microscope.

(b) Properties on Medium (28°C, cultured for 14 days)

| Medium | Growth | Aerial Hyphae | | Color or Substrate Hyphae | Soluble Pigment |
|---|---|---|---|---|---|
| | | Formation | Color | | |
| Sucrose Nitrate Agar | fair | fair | pale brown | pale yellowish brown | none |
| Glucose Asparagine Agar | " | none | — | " | " |
| Glycerin Asparagine Agar | " | slightly | white | " | " |
| Inorganic salt Starch Agar | good | good | pale reddish orange | " | " |
| Tyrosine Agar | " | fair | pale brown | " | " |
| Nutrient Agar | fair | none | —— | " | " |
| Yeast extract Malt extract Agar | good | good | pale reddish orange | " | " |
| Oatmeal Agar | " | " | " | " | " |
| Bennett's Agar | " | " | " | " | " |

The expression of color according to the "Guide to Color Standard" (Japan Color Institute ed.).

(c) Physiological properties

| | |
|---|---|
| Liquefaction of gelatin | poor growth |
| Production of melanoid pigment | negative |
| Tyrosinase reaction | negative |
| Peptonization of milk | positive |
| Coagulation of milk | negative |
| hydrolysis of starch | positive |

(d) Utilization of sugar

Growth in the presence of D-glucose, inositol
No growth observed in the presence of L-arabinose, D-xylose, D-fructose, sucrose, L-rhamnose, raffinose, D-mannitol

(e) Growth temperature

10°C No growth

28°C Favourable growth, abundant aerial hyphae

37°C Considerable growth, no formation of aerial hyphae and spores

42°C No growth

45°C No growth

50°C No growth

For the process of the present invention the above mentined strains PA—39504 and PA—31088 and their natural and artificial mutants capable of producing the antibiotic PA—39504—$X_3$ are used.

The fermentation process for producing PA—39504—$X_3$ may be carried out as follows. A PA—39504—$X_3$ producing strain is cultured on a nutrient medium under aerobic conditions, and after termination of cultivation PA—39504—$X_3$ is recovered from the culture broth.

In this fermentation, conventional medium components and conditions can be utilized. The medium includes substantially carbon sources, nitrogen sources and inorganic salts. If necessary, vitamins, precursors, and other components can be added to increase the yield of PA—39504—$X_3$. As carbon sources, for example glucose, starch, dextrin, glycerin, molasses, organic acid, etc. are used alone or in admixture. As nitrogen sources, for example soybean meal, corn steep liquor, meat extract, yeast extract, cotton seed meal, peptone, wheat germs, ammonium sulfate, ammonium nitrate, etc. are used alone or as a mixture. As inorganic salts, for example, calcium carbonate, sodium chloride, potassium chloride, magnesium sulfate, cobaltous chloride, various salts of phosphoric acid may, if necessary, be added to the medium.

The culture may be made according to methods generally used for the production of antibiotics. In the case of a liquid culture, particularly in large scale production, it may be effected under submerged aerated conditions. The pH in the medium is preferably 5.5—8.5. If the pH range in the medium varies during the production, a buffer such as calcium carbonate may be added to the medium. The cultivation is preferably conducted at 20 to 40°C, particularly 25 to 32°C. Although the cultivation time largely depends on the scale of the fermentation, it usually takes 20 to 80 hours in large scale production. If violent bubbling or foaming occurs during cultivation, defoamers such as vegetable oils, lard or a polypropylene glycol may be added before or during cultivation.

After termination of the cultivation, PA—39504—$X_3$ is recovered from the culture broth by conventional methods for recovering usual fermentation products. For example, filtration, centrifugation, adsorption-desorption procedures or chromatography with various ion exchange resins or other active adsorbents, extraction with various organic solvents, etc. can be employed in suitable combination. If necessary, any proper stabilizer may be added during the separation process to inhibit the decomposition of PA—39504—$X_3$.

If PA—39504—$X_3$ is produced with PA—39504—$X_1$ concurrently, PA—39504—$X_3$ can be separated from PA—39504—$X_1$ by chromatography using an ion exchange resin with properly

combined eluting solvents. Moreover, PA—39504—$X_3$ can be separated as a salt for the purpose of purification.

PA—39504—$X_3$ can be produced also by reduction of PA—31088—IV.

In reducing PA—31088—IV, mild conditions which have no influence on the other functional groups are employed. For example, reflux with triphenylphosphine in carbon tetrachloride, reaction with titanium trichloride, formation of an alkoxysulfonium salt followed by reduction with a borohydride or cyanogen borohydride, reaction with chromous chloride (Y. Akita et. al.: Synthesis, *1977*, 792), reaction with phosphorous pentoxide (I. W. J. Still et. al.: Synthesis, *1977*, 468), reaction with trifluoroacetic anhydride and hydrogen sulfide (J. Drabowicz et al.: Chem. Letters, *1977*, 767) can be utilized.

PA—39504—$X_3$ is active against gram-positive and gram-negative bacteria, and exhibits β-lactamase inhibition. The following indicate the antibacterial spectrum of PA—39504—$X_3$ and the antimicrobial activity against cephalosporinase type and penicillinase type β-lactamase producing organisms.

1. Antibacterial spectrum

| Organism tested | Minimum Inhibitory Concentration (μg/ml) |
|---|---|
| *Staphylococcus aureus 209P JC—1* | 6.3 |
| *Streptococcus pyogenes* C—203 | 0.8 |
| *Escherichia coli* NIHJ JC—2 | 0.8 |
| *Klebsiella pneumoniae* SRL—1 | 0.8 |
| *Klebsiella sp.* 363 (P) | 0.8 |
| *Proteus mirabilis* PR—4 | 1.6 |
| *Proteus vulgaris* CN—329 | 1.6 |
| *Enterobacter cloacae* 233 | 1.6 |
| *Pseudomonas aeruginosa* ATCC 25619 | 25 |

2. Antimicrobial activity against β-lactamase producing strains

| Organism tested | Minimum Inhibitory Concentration (μg/ml) |
|---|---|
| *Escherichia coli* 6 | 0.031 |
| *Proteus morganii* | 1 |
| *Proteus inconstans* 31 | 0.125 |
| *Enterobacter cloacae* 92 | 0.5 |
| *Proteus vulgaris* 31 | 0.25 |
| *Klebsiella sp.* 363 | 8 |
| *Enterobacter cloacae* 53 | 2 |
| *Escherichia coli* RTEM | 0.5 |
| *Escherichia coli* RGN 238 | 2 |

Note: Cephalosporinase type β-lactamase producing strains and penicillinase type β-lactamase producing strains are shown in the upper and lower colums, resp.

As shown in the above table, PA—39504—$X_3$ has activity against gram-positive and gram-negative bacteria, and particularly is effective against β-lactamase producing organisms, so that PA—39504—$X_3$ is useful as a drug and as a disinfectant. PA—39504—$X_3$ and its pharmaceutically acceptable salts are administered orally or parenterally to humans and animals. It may be administered orally in the form of tablets, capsules, powders, etc. together with e.g. excipients, stabilizers, preservatives, wetting agents, surfactants, or parenterally as an injection preparation, liniment or suppositories. The dosage of PA—39504—$X_3$ is variable according to therapeutic purposes, and in general it is effective at several tenths the dosage of cephalothin; for example, it may be administered subcutaneously at a dose of 0.1—30 g a day for an adult.

Moreover, PA—39504—$X_3$ is a potent β-lactamase inhibitor, so it can increase synergistically the antimicrobial activity of β-lactam antibiotics against β-lactamase producing organisms. Accordingly PA—39504—$X_3$ can be used together with known β-lactam antibiotics such as penicillin antibiotics (benzylpenicillin, phenoxymethyl penicillin, carbenicillin, ampicillin, amoxicillin, etc.) and cephalosporin antibiotics (cephaloridine, cephalothin, cefazolin, cephalexin, cefoxitin, cephacetrile, cefamandole, cephapirin, cepharadine, cephaloglycin, ceftezole, cefatrizine, cefmetazole, etc.).

In the following examples the production of PA—39504—$X_3$, the objective compound in the present invention is illustrated.

Example 1

A see culture of *Streptomyces argenteolus* PA—39504 (FERM—P No. 5265, ATCC No. 31589) is inoculated on 100ml of a medium consisting of soluble starch (0.5%), glucose (0.5%), polypeptone (0.5%), meat extract (0.5%), yeast extract (0.25%), sodium chloride (0.25%), deionized water (pH 7.0), placed in a 500 ml Sakaguchi flask, and cultured with shaking at a rate of 140 strokes per minute at 28°C for 48 hours. Four ml of the culture broth is transferred to 100 ml of a medium consisting of dextrin (2%), yeast extract (1%), tomato paste (2%), cobalt chloride (0.0005%), placed in a Sakaguchi flask, and cultured with shaking at 28°C for 72 hours.

Isolation

The culture broth obtained in the above procedure is filtered through Hyflosupercell®. The filtrate (800ml) is cooled to 10°C, adjusted at pH 7.0, passed through a Dowex® 1 x 2 column ($Cl^-$ type) (100 ml) at a flow rate of 10 ml per minute, and eluted with 3% sodium chloride in cold deionized water to give a PA—39504—$X_1$ fraction followed by elution with 3% sodium chloride in 50% methanol. The fractions (400 ml) that are positive to E. coli in the paper disc diffusion test are collected, adjusted to pH

7.0, and evaporated under reduced pressure to 150 ml. Then the resulting solution is applied to 150 ml Daiaion® HP—20 column and eluted at a flow rate of 10 ml per minute. An antimicrobially active fraction is eluted with water, followed by elution with cold deionized water containing 25% methanol to collect 50 ml of the other active fraction. The latter fraction is adjusted to pH 7.0, and then freeze-dried to give 15 mg of PA—39504—$X_3$ as a crude powder.

The powder (15 mg) is dissolved in a 5% methanol-0.05M sodium dihydrogenphosphate buffer (pH 7.0), subjected to high-performance liquid chromatography using a Nucleosil®-5—$C_8$ column (10 mm $\times$ 30 cm) with the above buffer. The active fraction is desalted and then freeze-dried. The resulting powder is again subjected to high-performance liquid chromatography using a Nucleosil®-7-$C_{18}$ column (10 mm $\times$ 30 cm) with a 10% methanol-0.5 M sodium dihydrogenphosphate buffer (pH 7.0), desalted, and freeze-dried to give PA—39504—$X_3$ as amorphous powder.

In order to obtain the same objective compound, the above fermentation and purification process can also be achieved with *Streptomyces tokunonensis* PA—31088 (FERM—P No. 4843, ATCC No. 31569) instead of *Streptomyces argenteolus* PA—39504 used in the above procedure.

Example 2

To 26 mg of titanium trichloride is added 26 ml of 0.5 M acetic acid-sodium acetate buffer (pH 7.0), and a solution of 20 mg of PA—31088—IV in 4 ml of the above buffer is adde thereto with stirring in a nitrogen atmosphere at room temperature. Ten minutes later, the reaction mixture is adsorbed on a 7 ml Daiaion® HP—20AG column previously treated with a 5% sodium chloride-0.05 M phosphate buffer, and after washing with 60 ml of the above buffer, eluted with water at a flow rate of 3 ml per minute. The antimicrobially active fraction (43 ml) is evaporated to about 3 ml under reduced pressure, adsorbed on a 18 ml Daiaion® HP—20AG column previously treated with water, and eluted with water. The resulting active fraction (28 ml) is evaporated under reduced pressure to about 2 ml, and then dried to give 5 mg of PA—39504—$X_3$ sodium salt as light yellow powder.

**Claims**

1. An antibiotic PA—39504—$X_3$ of the formula:

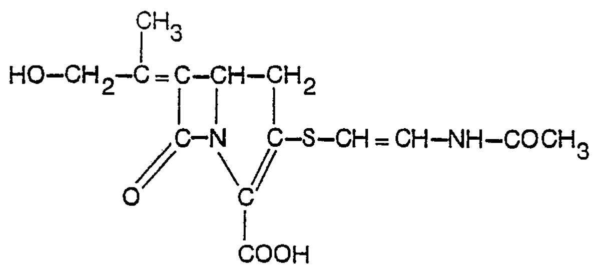

and pharmaceutically acceptable salts thereof, of which the sodium salt has the following properties:

(a) Ultraviolet absorption spectrum:
$\lambda_{max}^{H_2O}$ ($E_{1cm}^{1\%}$) nm 234(680), 296(316).

(b) Nuclear magnetic resonance spectrum
$\delta_{ppm}^{D_2O}$ (J = Hz), 1.96s3H, 2.05s3H, 3.09m2H, 4.21s2H, 4.85m, 6.01d1H (8.4), 7.13d1H (8.4).

(c) Circular dichroism spectrum:
$\lambda_{nm}[\theta]$ 425(O), 350(−2540), 332(−2190), 279(−10600), 263(O), 258(+3030), 252(O), 238(−10900), 224(O), 216.5(+8200), 211(+6060), 200(+23300).

(d) Mass spectrum (Field desorption mass spectrum)
M/z 459 = [$M^+$] (measured as p-nitrobenzyl ester)

2. A process for producing the antibiotic PA—39504—$X_3$ according to claim 1, which comprises cultivating *Streptomyces argenteolus* PA—39504 or *Streptomyces tokunonensis* PA—31088 or PA—39504—$X_3$ producing mutants thereof under aerobic conditions and isolating the accumulated antibiotic PA—39504—$X_3$ from the culture broth and, if desired, converting it into a pharmaceutically acceptable salt by treatment with a base.

3. The process claimed in claim 2, wherein *Streptomyces argenteolus* PA—39504 is cultivated.

4. The process claimed in claim 2, wherein *Streptomyces tokunonensis* PA—31088 is cultivated.

5. The process as claimed in claim 2, wherein the cultivation is effected at 20 to 40°C for 20 to 80 hours.

6. A process for producing the antibiotic PA—39504—$X_3$ according to claim 1 which comprises reducing the antibiotic PA—31088—IV and, if desired, converting it into a pharmaceutically acceptable salt by treatment with a base.

7. Pharmaceutical compositions comprising the antibiotic PA—39504—$X_3$ or its pharmaceutically acceptable salt according to claim 1.

**Patentansprüche**

1. Ein Antibiotikum PA—39504—$X_3$ der Formel

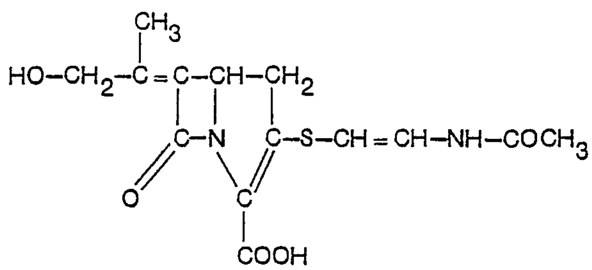

und seine pharmakologisch verträglichen Salze, wobei das Natriumsalz folgende Eigenschaften hat:

(a) UV—Absortionsspektrum:
$\lambda_{max}^{H_2O}$ ($E_{1cm}^{1\%}$) nm 234(680), 296(316).

(b) NMR—Spektrum:
$\delta_{ppm}^{D_2O}$ (J = Hz), 1.96s3H, 2.05s3H, 3.09m2H, 4.21s2H, 4.85m, 6.01d1H (8.4), 7.13d1H (8.4).

(c) Circulardichroismus-Spektrum:
$\lambda_{nm}[\theta]$ 425(O), 350(−2540), 332(−2190), 279(−10600), 263(O), 258(+3030), 252(O), 238(−10900), 224(O), 216.5(+8200), 211(+6060), 200(+23300).

(d) Massenspektrum (Felddesorptions-Massenspektrum):
M/z 459 = [$M^+$] (bestimmt als p-Nitrobenzylester)

2. Verfahren zur Herstellung des Antibiotikums PA—39504—$X_3$ nach Anspruch 1, dadurch gekennzeichnet, daß man Streptomyces argenteolus PA—39504 oder Streptomyces tokunonensis PA—31088 oder PA—39504—$X_3$ bildende Mutanten davon unter aeroben Bedingungen züchtet und das sich in der Kulturbrühe anreichernde Antibiotikum PA—39504—$X_3$ isoliert und gegebenenfalls durch Umsetzung mit einer Base in ein pharmakologisch verträgliches Salz überführt.

3. Verfahren nach Anspruch 2, dadurch gekennzeichnet, daß man Streptomyces argenteouls PA—39504 züchtet.

4. Verfahren nach Anspruch 2, dadurch gekennzeichnet, daß man Streptomyces tokunonensis PA—31088 züchtet.

5. Verfahren nach Anspruch 2, dadurch gekennzeichnet, daß man die Züchtung bei 20 bis 40°C während 20 bis 80 Stunden durchführt.

6. Verfahren zur Herstellung des Antibiotikums PA—39504—$X_3$ nach Anspruch 1, dadurch gekennzeichnet, daß man das Antibiotikum PA—31088—IV reduziert und gegebenenfalls durch Umsetzung mit einer Base in ein pharmakologisch verträgliches Salz überführt.

7. Arzneimittel, enthaltend das Antibiotikum PA—39504—$X_3$ oder dessen pharmakologisch verträliches Salz gemäß Anspruch 1.

**Revendications**

1. Antibiotique PA—39504—$X_3$ caractérisé en ce qu'il est représenté par la formule:

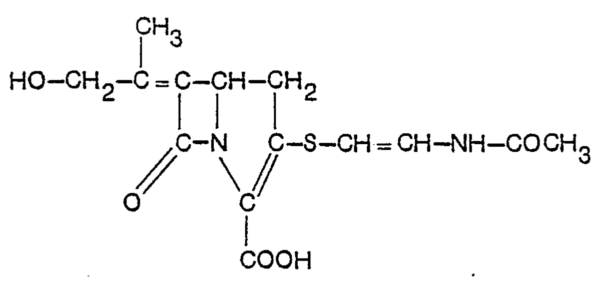

et ses sels acceptables du point de vue pharmaceutique, par mi lesquels le sel sodique présente les properiétés suivantes:

(a) Spectre d'absorption du rayonnement ultraviolet:
$\lambda_{max}^{H2O}$ ($E_{1cm}^{1\%}$) nm 234(680), 296(316).

(b) Spectre de résonance magnétique nucléaire:
$\delta_{ppm}^{D2O}$ (J = Hz), 1.96s3H, 2.05s3H, 3.09m2H, 4.21s2H, 4.85m, 6.01d1H (8.4), 7.13d1H (8.4).

(c) Spectre de dichroïsme circulaire:
$\lambda_{nm}[\theta]$ 425(O), 350(−2540), 332(−2190), 279(−10600), 263(O), 258(+3030), 252(O), 238(−10900), 224(O), 216.5(+8200), 211(+6060), 200(+23300).

(d) Spectre de masse (spectre de masse par désorption dans un champ):
M/z 459 = [$M^+$] (mesuré sous forme de l'ester p-nitrobenzyle).

2. Procédé de préparation de l'antibiotique PA—39504—$X_3$ selon la revendication 1, caractérisé en ce que l'on cultive du *Streptomyces argenteolus* PA—39504 ou du *Streptomyces tokunonensis* PA—31088 ou des mutants produisant du PA—39504—$X_3$ de ceux-ci dans des conditions aérobies et que l'on isole l'antibiotique PA—39504—$X_3$ accumulé à partir de ce bouillon de culture et, si cela est désiré, on convertit celui-ci en un sel acceptable du point de vue pharmaceutique en le traitant avec une base.

3. Procédé selon la revendication 2, caractérisé en ce que l'on cultive le *Streptomyces argenteolus* PA—39504.

4. Procédé selon la revendication 2, caractérisé en ce que l'on cultive le *Streptomyces tokunonensis* PA—31088.

5. Procédé selon la revendication 2, caractérisé en ce que la culture est effectuée à 20 à 40°C pendant 20 à 80 heures.

6. Procédé de préparation de l'antibiotique PA—39504—$X_3$ selon la revendication 1, caractérisé en ce que l'on réduit l'antibiotique PA—31088—IV et, si cela est désiré, on convertit celui-ci en un sel acceptable du point de vue pharmaceutique en le traitant avec une base.

7. Compositions pharmaceutiques caractérisées en ce qu'elles comprennent l'antibiotique PA—39504—$X_3$ ou son sel acceptable du point de vue pharmaceutique selon la revendication 1.

Fig. 1

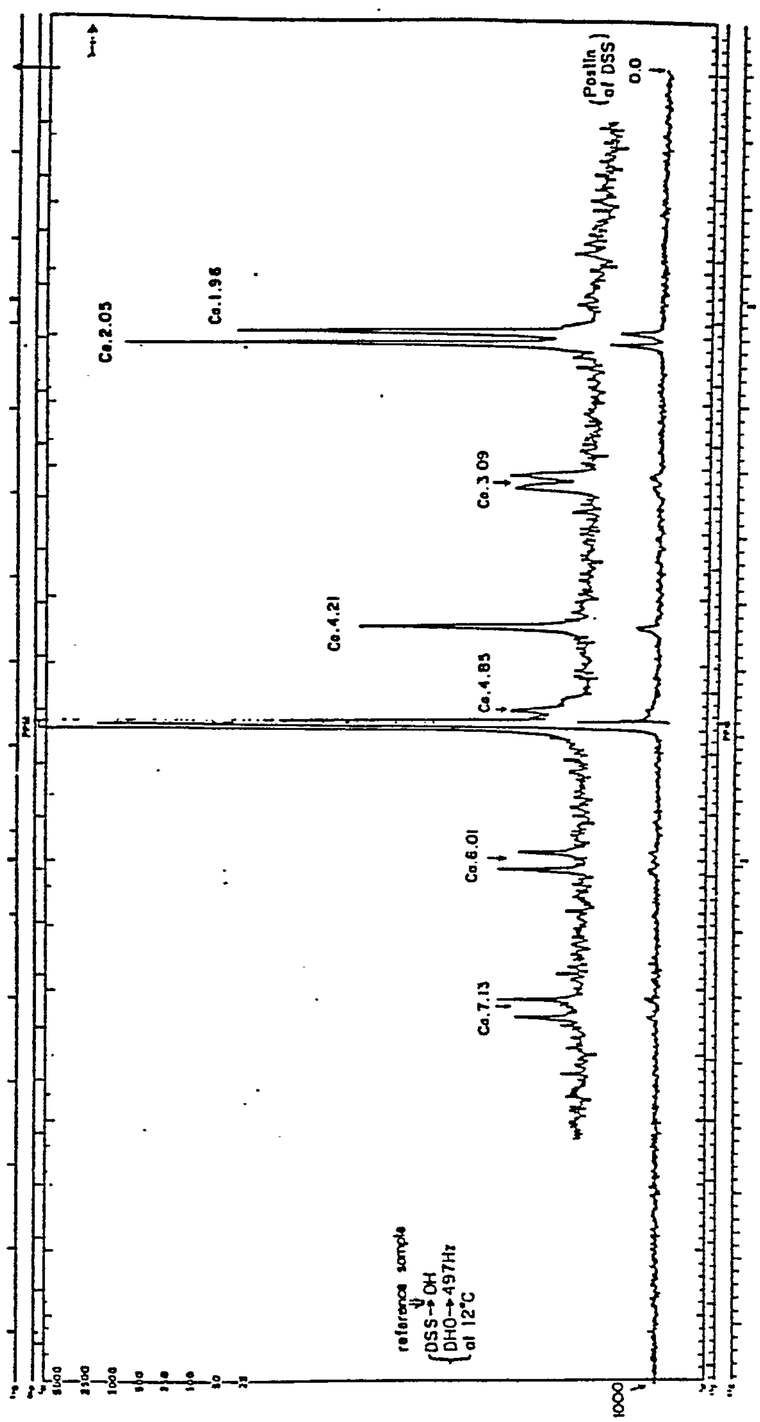

Ca.2.05
Ca.1.96
Ca.3.09
Ca.4.21
Ca.4.85
Ca.6.01
Ca.7.13
(Position of DSS)
0.0
reference sample
DSS→OH
DHO→497Hz
at 12°C
1000